(19) Europäisches Patentamt / European Patent Office / Office européen des brevets

(11) **EP 0 938 338 B1**

(12) **EUROPEAN PATENT SPECIFICATION**

(45) Date of publication and mention
of the grant of the patent:
**30.09.2009  Bulletin 2009/40**

(21) Application number: **97936687.9**

(22) Date of filing: **01.08.1997**

(51) Int Cl.:
*A61K 47/10* (2006.01)   *A61K 47/12* (2006.01)
*A61K 47/22* (2006.01)

(86) International application number:
**PCT/EP1997/004273**

(87) International publication number:
**WO 1998/005359 (12.02.1998 Gazette 1998/06)**

(54) **CARRIER COMPOSITION FOR INFLUENCING THE TIGHT JUNCTION PERMEABILITY**

TRÄGER ZUR BEEINFLUSSUNG DER DURCHLÄSSIGKEIT DER DICHTEN VERBINDUNGSSTELLEN

COMPOSITION DE TRANSPORT DESTINEE A INFLUENCER LA PERMEABILITE DES JONCTIONS SERREES

(84) Designated Contracting States:
**CH DE ES FR GB IE LI**

(30) Priority:  **02.08.1996  EP 96202178**

(43) Date of publication of application:
**01.09.1999  Bulletin 1999/35**

(73) Proprietors:
• **Ghyssaert, Joris Rosa Michael**
  **2018 Antwerpen (BE)**
• **Verhaeren, Edward Henri Charles**
  **2640 Mortsel (BE)**

(72) Inventor: **VERHAEREN, Edward, Henri, Charles**
**B-2640 Mortsel (BE)**

(74) Representative: **De Clercq, Ann G. Y. et al**
**De Clercq, Brants & Partners c.v.**
**Edgard Gevaertdreef 10a**
**9830 Sint-Martens-Latem (BE)**

(56) References cited:
WO-A-94/00155     WO-A-95/03823
WO-A-96/13226     US-A- 4 312 856
US-A- 4 970 233

• E.H.C. VERHAEREN ET AL.: "THE EFFECT OF 2,4,6-TRIAMINOPYRIMIDINE ON INCREASED MUCOSAL PERMEABILITY OF GUINEA-PIG COLONIC MUCOSA, FOLLOWING ADMINISTRATION OF 1,8-DIHYDROXYANTHRAQUINONE, SALICYLIC ACID AND DINITROPHENOL" PHARMACEUTISCH WEEKBLAD SCIENTIFIC EDIDITION, vol. 3, no. 1, 20 February 1981, UTRECHT (NL), pages 815-819, XP002022516
• B.J. AUNGST ET AL.: "enhancement of the intestinal absorption of peptides and non-peptides" JOURNAL OF CONTROLLED RELEASE, vol. 41, no. 1,2, 1 August 1996, AMSTERDAM (NL), pages 19-31, XP000592980
• K.L. LUTZ ET AL.: "molecular structure of the apical junction complex and its contribution to the paracellular barrier" JOURNAL OF PHARMACEUTICAL SCIENCES, vol. 86, no. 9, September 1997, WASHINGTON (US), pages 977-984, XP002047661

**Description**

The present invention relates to a carrier composition for influencing the tight junction permeability, in particular for transporting naturally unresorbable substances into the human or animal body. The invention further relates to the use of various substances in the transportation of these substances.

Life in its essential form involves the presence and the maintenance of a complex compartmentalisation. Substrates should only react with enzymes on the right moment, ATP (adenosine triphosphate) should be produced after demand. Moreover, a strict isotonic medium is necessary. Therefore not only cell membranes are highly sophisticated structures physically and chemically, also mucosal tissues exhibit a fascinating complexity. The first aim of specific mucosal structures is to maintain carefully the internal equilibrium versus the hostile surrounding.

In most parts of the body adjacent cells are thus sealed together to prevent the passage of fluids through the cell layer. Seals are normally formed between the epithelial cells that line all the cavities of the mammalian body. The impermeable junctions, termed "tight junctions" or "zona occludens", seal off, for example, the lumen of the intestine. Tight junctions are composed of thin bands that completely encircle a cell and are in contact with thin bands of adjacent cells. It is known that these junctions are impermeable to salts and even water.

Nutrients are absorbed from the intestine into one side of the epithelial cell and then released from the other side because the tight intercellular junctions do not even allow small molecules to diffuse directly from the intestinal lumen into the blood. This transport through the cell is called "transcellular".

Biologically active substances, in particular pharmaceuticals, can be administered to the human and animal body through various routes, such as through intramuscular or intravenous injection, orally, rectally etc.. Between these routes oral or rectal administration is usually preferred because these are most convenient for the patient and do not require the intervention of a physician. However, many pharmaceutical substances are not resorbable in the digestive tract. The transcellular route is closed for large and charged molecules. Furthermore, some other substances, like antibiotics are resorbed (or not) in an unpredictable manner. Obviously, these types of molecules cannot pass the tight junctions either.

A complicating factor moreover concerning oral administration is the assessment that many substances are degraded in the stomach. Enteric coatings can protect these degradable substances, without however influencing their resorbability in the intestine after a successful pass the stomach. Non resorbable substances are usually not resorbed in the rectum either.

It is therefore an object of the invention to provide for a possibility to transport naturally non-resorbable substances into the human or animal body. A further object is to provide a possibility of transporting substances out off the body.

Extensive research led to the finding that the tight junctions in the body may be actively opened to let substances in. Opening of tight junction per se is not new and may be observed for example in the case of diarrhoea, where sodium and water move from the blood into the colon driven by the hydrostatic pressure thereof. Sometimes tight junctions also open to let substances in to maintain the intracellular solute composition constant. However, in these cases opening of the tight junctions is induced by the body itself and cannot be influenced from the outside. The present invention now makes an active opening of the tight junctions possible. Transport through the tight junctions is called "paracellular".

The invention is based on the insight that the opening is for example achieved upon so-called "uncoupling" of the mitochondria. Uncoupling is a phenomenon that leads to a decrease or even termination of ATP production in the mitochondria. This uncoupling was now found to contribute to the opening of the tight junctions. The effect is only temporarily and the tight junctions close again.

The invention now provides for a carrier composition for transporting (biologically active) substances into the human or animal bod, as defined in the claims.

Although the exact cause of the opening is not yet identified it seems probable that keeping the junctions tight is an energy requiring process. Lack of ATP will thus lead to leakage of the tight junctions. Therefor, inhibiting ATP production through other means than mitochondrial uncoupling, for example by inhibiting electron transport, will also result in an opening of the tight junctions. In this application the term "(mitochondrial) uncoupler" is intended to encompass every type of substance that is capable of opening the tight junction.

The advantage of the present invention is that the opening of the tight junctions can be controlled and is completely reversible. No damage to the cells or their surroundings was observed.

Suitable mitochondrial uncouplers are for example the non steroidal anti inflammatory drugs (NSAIDS) salicylic acid and its different salts, aryl acetic acid derivatives, aryl propionic acid derivatives, indole derivatives, fenamates, oxicams and phenylbutazone, or the polyphenols like dihydroxyanthraquinones, oxyphenisatin, phenolphthalein, or bile salts and their derivatives, as well as several oils or fatty acids, like ricinus oil or ricinoleic acid. Generally most organic compounds exerting a laxative effect can be employed. These include but are not limited. Salicylates, diflunisal, fenoprofen, calcium, flurbiprofen, ibuprofen, indomethacine, indomethacin sodium trihydrate, ketoprofen, meclofenamate, mefenamic acid, naproxen, naproxen sodium, oxyphenbutazone, phenylbutazone, piroxicam, sulindac and tolmetin sodium. This list is given as an example only, and does not limit the invention. The skilled person will be capable to select suitable uncouplers based on the disclosure given herein.

Although the sole opening of the tight junctions may be sufficient for some applications, for example for application of the carrier composition on the skin, usually an additional "driving force" is preferred to control the direction of transport of the (biologically active) substance. One preferred driving force is an osmotic solution, such as a hypotonic solution, like bi-distilled water, for transport into the body. Another example is hyaluronidase. Driving forces are particularly preferred for rectal, vaginal, sublingual, nasal and oral administration forms, although transdermal transport is also accelerated by it.

Biologically active substances that might be incorporated in the carrier composition are preferably substances having a molecular weight of at least 300, preferably at least about 1,000, because these cannot be resorbed otherwise. Examples of these substances are peptide hormones, such as calcitonin, secretin, vasopressin, insulin, angiotensin, felypressin, thyrotropin releasing hormone, gonadotropin releasing hormone, corticotropin, prolactin, somatotropin, thyrotropin, luteinizing hormone (LH), callicrein, parathyrin, glucagon, oxytocin, gastrin and serum gonadotropin, or polysaccharides, such as heparin sodium, heparin calcium and chondroitin sulfate or cosmetically active substances, like collagen, etc. Generally the invention is suited for all drugs that are unpredictably resorbed.

The carrier composition may be used for therapeutic, diagnostic, prophylactic and cosmetical applications.

Diagnostic applications may be the transportation into the body of labeled compounds that may be targeted to a specific site to be diagnosed or the extraction of substances to be diagnosed. Prophylactic applications encompass for example vaccines. Therapeutical and cosmetical applications are numerous and considered self-evident.

Cosmetical compositions will usually be intended for transdermal administration by application to the skin. The other types of compositions may in particular be used for rectal, vaginal, sublingual, nasal and also transdermal administration. Rectal and vaginal administration forms may for example take the form of hollow suppositories in which the (liquid) carrier composition is incorporated. Preferred sublingual administration forms are pills, tablets, coated oral administration or pills with retarded release. For nasal administration aerosols, sprays and nose drops are particularly suitable. Oral administration forms encompass for example hollow capsules, liquid medicines, sublingual tablets. For transdermal and or transmucosal administration e.g. transdermal pads, unguents, gels, ointments, fluids, oils, etc. are particularly preferred.

The invention further relates to the use of uncoupling agents for influencing the permeability of tight junctions, and in the preparation of the carrier compositions.

The invention also relates to the use of the carrier compositions for the diagnostic, prophylactic, therapeutic or cosmetic treatment of the human or animal body.

The invention will be further explained in the following examples that are given for illustration purposes only and are in no way intended to be limiting to the invention.

## EXAMPLES

### EXAMPLE 1

Influence of mitochondrial uncouplers on paracellular permeability

### 1. Introduction

Enhanced mucosal permeability reflects for a laxative effect. Therefore laxative anthraquinones must alter mucosal permeability. This principle was used to show that a non resorbable radio-active labelled molecule $^{99m}$Tc-EDTA (Metastable technetium - Ethylene Diamine Tetra Acetic acid) could be introduced into the body of the rat by addition of a laxative, which is a mitochondrial uncoupler.

The present inventor investigated the influence of 1,8-dihydroxyanthraquinones, salicylic acid and dinitrophenol (DNP), all mitochondrial uncouplers, on paracellular permeability with the aim to find a causal connection between a mitochondrial uncoupling effect and an influence on permeability.

### 2. Materials and methods

### 2.1. Operation procedure

Guinea pigs of either sex (250-280 g) were fed for one week with pelleted food containing 0.1% 1,8 dihydroxyanthraquimone. For this purpose the necessary amount of Anthraquinone was solved in ether and sprayed over the pelleted foods. The ether was evaporated. The calculated daily administration of 1,8 dihydroxyantraquinone was 20 mg.

The animals were fasted 6 hours before the experiment in order to empty the proximal colonic segment.

Anaesthesia was induced by peritoneal injection of ethyl urethane (0.9 g.kg$^{-1}$) and body temperature was maintained at 37°C by a thermostatically controlled heated table.

A midventral abdominal incision was made and the large intestine exposed.

The proximal colon was located and a segment was ligated with a length of 7 branches of the superior mesenteric vein to obtain a physiological length of tissue.

In this ligated colon segment 0.25 ml $^{99m}$TcEDTA was injected. Immediately the abdominal cavity was closed.

The biodistribution of the labelled complex was visualised by a γ-camera Pho/GAMMA®HP-Nuclear; Chicago fitted with a low energy (240 keV) convergating collimator.

After one hour the guinea pigs were killed with chloroform and both kidneys, the ligated colon segment and the liver were isolated. Urine was collected from the urine bladder using a syringe. The activity of the samples was measured by a Gamma-counter (Berthold, Gamma-sample changer BF 5300) positioned on 140 $\pm$ 10 keV.

In order to determine the influence of the salicylic acid and dinitrophenol on mucosal permeability, 0.25 ml of salicylic acid was injected in the ligated colon segment of untreated animals. After an exposure time of 30 minutes the labelled $^{99m}$Tc-EDTA-complex was injected. The procedure continued as described for 1,8 dihydroxyantraquinone.

2.2. Preparation of the $^{99m}$TC-EDTA-complex

The instant kit method of Eckelman and Richards (J. Nucl. Med. 11, 761(1970)) was modified. 5.0 mg of Na$_2$EDTA and 0.5 mg of snCl$_2$.2H$_2$0/ml was filtered through a 0.22 $\mu$ membrane filter and conserved in 3 ml vacuum serum tubes. The kits were kept at -20°C.

Prior to the experiment 1 ml sterile eluate of a $^{99m}$Tc-generator (Sterow™ 99m Byk-Malincrodt) with an activity of 2-10 $\mu$ Ci was added to the thawed kit. After mixing the efficiency of the complex formulation was assessed by paper chromatography (Whatman N° 1) using physiological water and methylethylketone as mobile phase. Detection was made with a Berthold Scanner. More than 98% of the present $^{99m}$Tc was complexed. The EDTA-complex does not attribute to the physiological osmolarity as measured with an Osmette Precision Osmometer (Precision Systems, Mass.).

2.3. Preparation of the salicylic acid and the dinitrophenol

100 mg salicylic acid or dinitrophenol was solved in bidistilled water. Sodium hydroxide N was added until complete dissolution. pH was adjusted on 8.5. Water was added to a volume of 10 ml. Osmolarity was adjusted (275 mOsm/1) by adding 23 and 14 mg sodium chloride respectively.

3. Results

In ligated colon segments only a minor fraction of the enclosed radioactivity migrates from the gut lumen to the blood. While the chemical complex is strongly hydrophilic, it is easily filtered by the kidneys and deposited in the urine bladder.

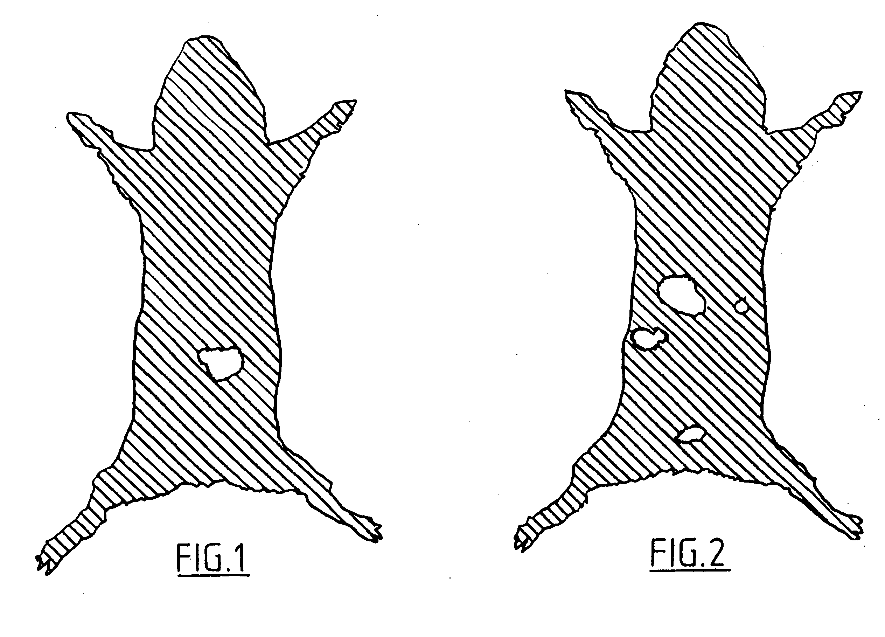

Fig. 1 of an untreated animal clearly shows that after one hour all radioactivity remains in the ligated colon segment proving the impermeability of colonic epithelium. The photo was corrected for the body contour allowing an easy location of the radioactive sites.

Fig. 2 shows an animal treated with 1,8-dihydroxyanthraquinone during one week. It is obvious that a remarkable increase of colonic permeability is noted. As a consequence both kidneys and the urine bladder are clearly visible.

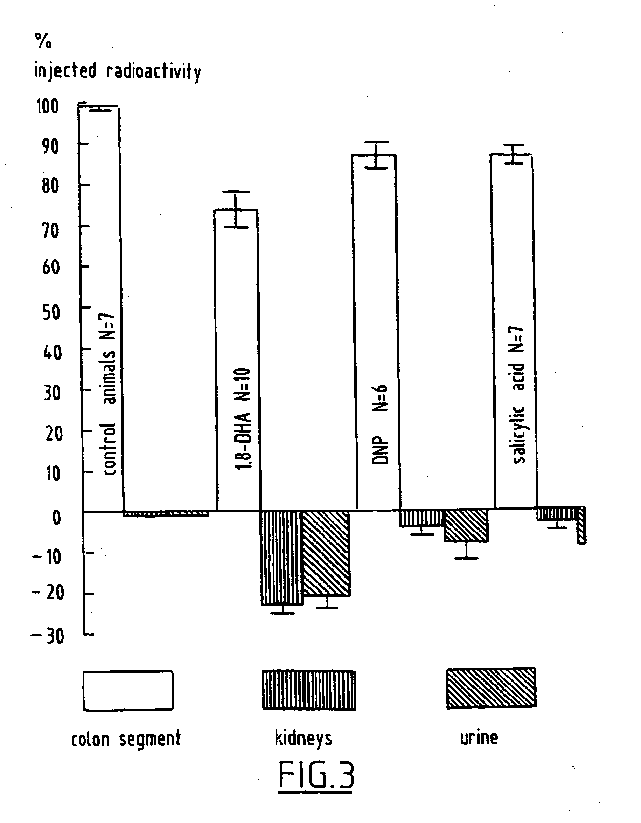

One hour after the administration of the radioactive complex nearly 25 % of the radioactivity has leaked from the colon and concentrated in the urine bladder. Fig. 3 shows the distribution of the administered radioactivity in terms of percentages. The mean (X) and the standard deviation (SD) are displayed.

A similar, although less pronounced effect was noted after the administration of salicylic acid and dinitrophenol. It was found that this was due to a systemic effect rather than a local one. All differences are statistically significant (p < 0.01). High resolution images of the Scanning Electron Microscope do not reveal any trace of mucosal scrape-off, nor any other serious tissue damage. The observed effects proved to be fully reversible and were electron microscopically not able to explain the observed enhanced permeability.

4. Conclusion

It was found that non-resorbable $^{99m}$Tc-EDTA, if injected in a colon segment, still remains in that segment after one hour. On the other hand, if a small amount of mitochondrial uncoupler (anthraquinone, salicylate, dinitrophenol) is added, the complex is readily resorbed entering the bloodstream, filtered by the kidneys and concentrated in the urine bladder.

Thus, the transfer from lumen to blood of a virtually non-resorbable test molecule as $^{99m}$Tc-EDTA is significantly enhanced after previous administration of the mitochondrial uncouplers 1,8-dihydroxyanthraquinone, salicylic acid and dinitrophenol.

**EXAMPLE 2**

The influence of protonated TAP on increased mucosal permeability, hollowing a 1,8-dihydroxyanthraguinone administration

1. Introduction

While transmucosal tissue resistance significantly changes by addition of mitochondrial uncouplers and while this resistance principally accounts for the paracellular route, experiments were carried out in order to identify the resorption route.

$^{99m}$Tc-EDTA is not able to migrate through an intact mucosa. While this transfer is possible in 1,8-dihydroxyanthraquinone pre-treated animals, this phenomenon can only be explained by the fact that transcellular transport is facilitated by anthraquinone or that the transport is not trans- but paracellular by way of the tight junctions.

2,4,6-Triaminopyrimidine (TAP) was used to block the paracellular way. If the resorption of a non resorbable molecule $^{99m}$Tc-EDTA complex was enhanced by the addition of a mitochondrial uncoupler and blocked by TAP, then the route followed by the $^{99m}$Tc-EDTA complex was unequivocally identified as the paracellular one.

2. Materials and methods

The same materials and methods were used as described in Example 1. However, instead of $^{99m}$c-EDTA TAP was used followed by $^{99m}$Tc-EDTA.

Preparation of the TAP-solution

100 mg TAP was solved in bidistilled water. The pH was adjusted to 6.0 with HCl 2 N and completed to 10.0 ml. Final concentration was 80 mM. To obtain a physiological osmolarity 16 mg sodium chloride was added.

3. Results

Electron microscopical examination showed that transcellular transport is not facilitated by anthraquinone. The remaining alternative was assessed by evaluating the influence of TAP on the permeability of the paracellular route.

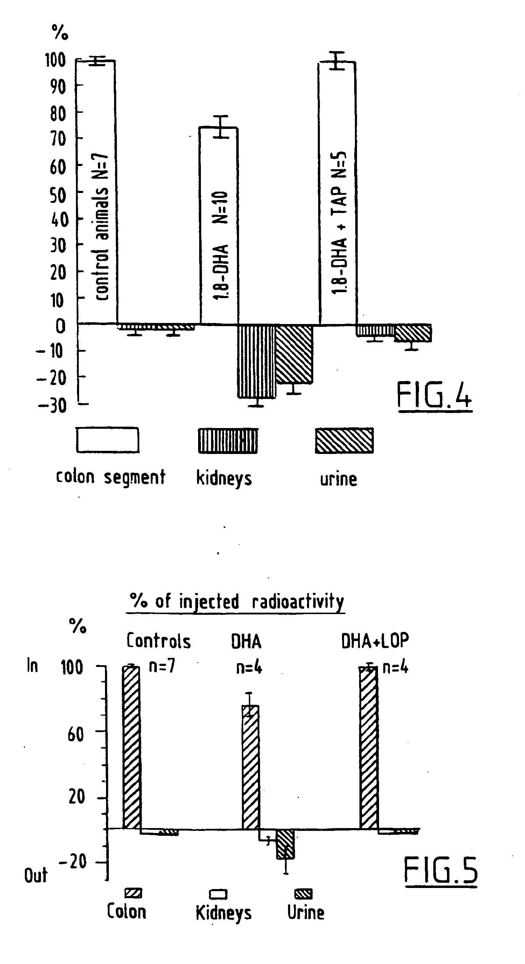

The measured mucosal permeability following a 1,8 -dihydroxyanthraquinone administration is nearly completely restored by the action of TAP (Fig. 4). The recorded permeability changes are statistically significant (p < 0.01) resulting in the conclusion that $^{99m}$Tc-EDTA follows the same paracellular route as urea and glycerol,

4. Conclusion

1,8-dihydroxyanthraquinone and most probably all other anthraquinone derivatives cause changes in the permeability of the mucosal tight junctions. This possibly could be the consequence of an ATP deficiency. Anyway, the effect of anthraquinones can be situated at the tight junctions as demonstrated by the TAP experiments.

**EXAMPLE 3**

Blockade of the paracellular route by anti-diarrhoeal drugs

A similar experiment was performed with the anti-diarrhoeal drugs loperamide, morphine and gallotannins with similar results.

Fig. 5 shows the antagonistic activity of loperamide on the activity of 1,8-dihydroxyanthraquinone (DHA). Also the effect of anthraquinone is reversible. When the antidiarrhoeal agent loperamide is administered in combination with DHA, the junctions of the colonic mucosa "close" again. The initial impermeability of the intestinal wall is restored and the $^{99m}$Tc-EDTA complex is no longer transferred from lumen to blood.

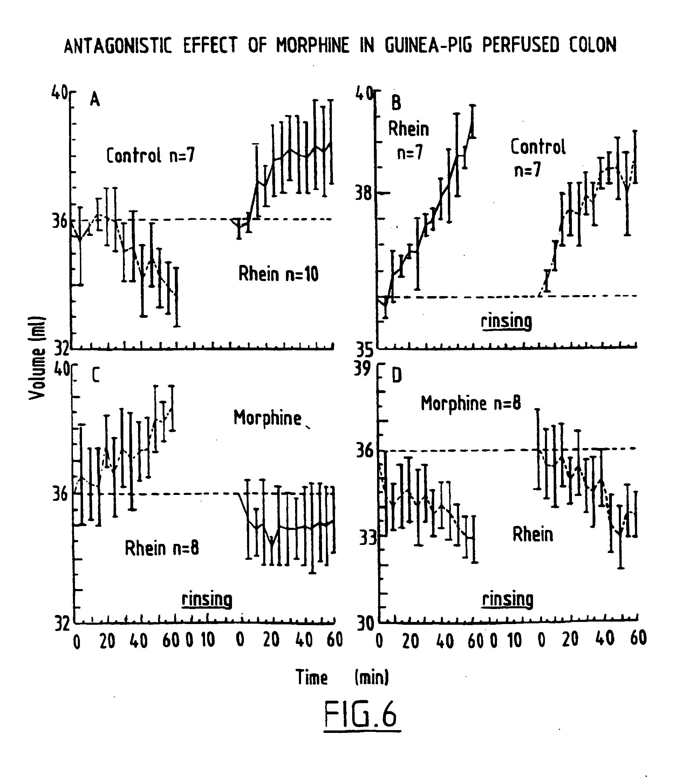

Fig. 6 shows that morphine also reverses the effect_of the anthraquinone rhein which opens the tight junctions resulting in secretion of water in the colon. If morphine was perfused after rhein, the opiate drug counteracted the secretory effect of rhein. The differences are statistically significant for P <0.05 using the paired t-test. Prior perfusion with morphine even protects the large intestine from the secretory effect of rhein. $^{99m}$Tc-EDTA complex did reach the bloodstream but there were differences in absorption rate as morphine retained nearly all the radioactive compound in the colon, while rhein facilitated significantly its transport from the colon through the mucosal barrier into the blood.

On the other hand, rhein was able to reestablish the absorption rate of the $^{99m}$Tc-EDTA. Morphine counteracted the inhibition of sodium absorption caused by rhein. Even more relevant was the significant rise in K+ concentration under the influence of rhein. This effect was completely antagonised by morphine.

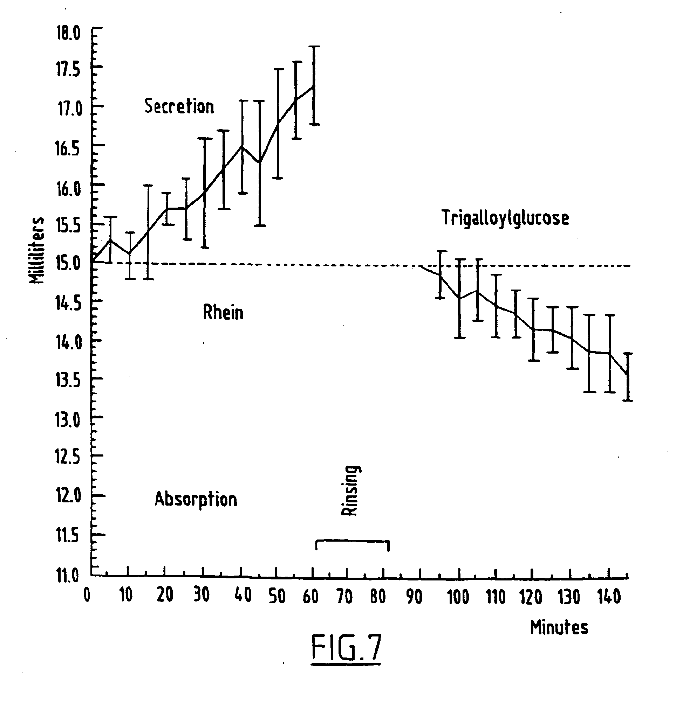

The antagonising effect of trigalloylglucose on rhein is shown in Fig. 7. Similar results are obtained by a substitution of trigalloylglucose by pentagalloylglucose. Both molecules inhibit completely the secretory effect of rhein. Due to the similarity of both curves, the result of pentagalloylglucose is not displayed.

Moreover, it was found that the permeability increase due to uncouplers is fully reversible. Even the electron - microscopically observed tissue abnormalities after chronic intoxication with anthraquinones where reversible.

It should be noticed that in this experiment the administrated amount exceeds the normal doses by a factor 500.

## EXAMPLE 4

The antagonistic effect of Loperamide on the mitochondrial uncoupling effect of 1,8-dihydroxyanthraquinone

### 1. Introduction

The link between permeability and mitochondrial uncoupling was also demonstrated, while in vivo experiments demonstrate that isolated mitochondria were effectively protected by Loperamide against the uncoupling effect of anthraquinones. This effect was assessed by chemiluminescent techniques as Luciferine - Luciferase.

### 2. Materials and methods

### 2.1. Isolation Procedure of Mitochondria

Mitochondria were isolated from Phaseolus aureus. Beans were allowed to sprout at 28°C during 5 days in the dark. 40 g sprouts were used.

The plant material was homogenised at 3°C with a solution of 0.3M Mannitol, 1 mM $Na_2$EDTA, 0.1% BSA, 0.05% Cysteine. The pH was adjusted to 7.2

During homogenisation 6 N KOH was added to keep the pH at 7.2.

The suspension was centrifuged at 2°C (2000 x g for 15 minutes). The pellet was removed and the supernatant centrifuged again during 20 minutes (8000 x g). The pellet was carefully resuspended in 1 ml of a solution (reaction medium) with 0.3M Mannitol, 10mM phosphate buffer, 10mM KCl, 5mM $MgCl_2$, 0.1% BSA. The pH was adjusted at 7.2.

Mitochondrial quality was assessed by measuring the formation speed of ATP following a ADP injection. ATP was measured by L-Luciferin-Luciferase light emission in the LKB-Luminometer.

### 2.2. ATP-measurements

The measurement of ATP was carried out in a medium consisting of a 1/1 mixture of reaction medium and Luciferin-Luciferase buffer. The complex composition is :

- 200 $\mu$l Luciferin-Luciferase solution
- 400 $\mu$l TRIS.HCl 0.05 M - EDTA 2 mM pH 7.75
- 400 $\mu$l reaction medium as explained above
- 20 $\mu$l mitochondrial suspension
- 10 $\mu$l Succinate 10 mM pH 7.7
- 20 $\mu$l Loperamide HCl (5 mg/80 ml water) or water depending on the experimental set up

1,8 dihydroxyanthraquinone was used in a concentration of 2 mg/100 ml methanol.

### 3. Results

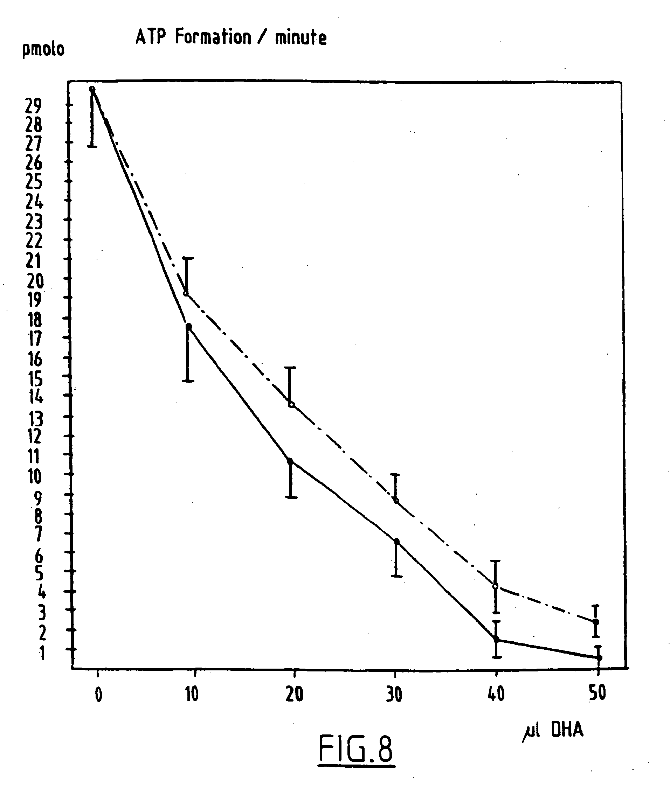

Fig. 8 shows the influence of increasing concentrations DHA (dihydroxyanthraquinone) on the generation speed of ATP

o: in the absence of loperamide
o: in the presence of loperamide
N= 15

Displayed are the Mean (X) and 2x SD (standard deviation).

The obtained results confirm that loperamide HCl protects mitochondria in vitro against the uncoupling effect of anthraquinones.

This finding strongly suggests that the observed permeability changes after DHA administration are at least partly due to an effect on mucosal mitochondria.

To collect significant results a possible interference of Methanol and Loperamide HCl on the light generating Luciferin-Luciferase enzymcomplex could not be excluded.

## EXAMPLE 5

The effect of anthraquinones and loperamide on temperature increase in the ligated rat colon segment

### 1. Introduction

It was demonstrated in vivo that mitochondrial uncoupling of the mucosal tissue of the intestine, locally results in a significant temperature increase, even 0,9°C above body temperature, due to the dissipation of heat by the mitochondria (while energy is never lost) and reflecting the energy content of the non-produced ATP-molecules.

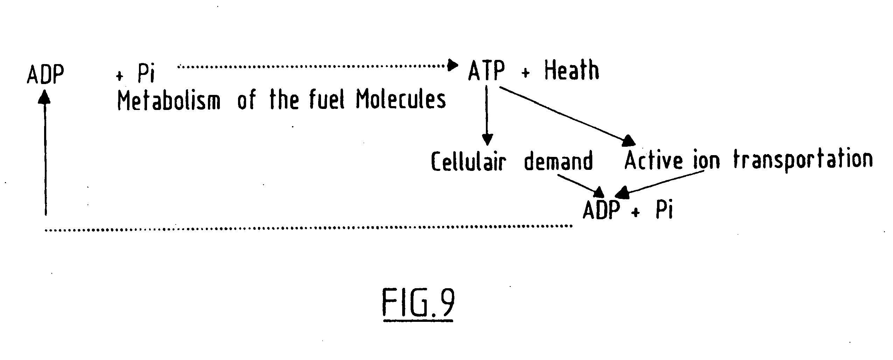

In the living cell ATP is generated from ADP by means of coupled fosforylation reactions. The turn-over of ATP is schematically visualised in Fig. 9. In the biochemical production process of ATP nearly 38 to 44% of the free energy of the fuel molecules (FADH2, NADH, succinate) is materialised under form of ATP. The remaining free energy fraction is dissipated as heat. This heat production, necessary for the isothermal steady state of the organism, is expected to rise considerably as a consequence of mitochondrial uncoupling, because then the energy normally incorporated in ATP is also available and dissipated as heat. Therefore it should be possible to measure the energy equilibrium during oxidative fosforylation by following the heat production.

### 2. Materials and methods

#### 2.1. Operation procedure

While animals are thermally unstable during narcosis special precautions had to be taken to overcome this problem. Guinea pigs (400 g, males) were fasted 12 hours before the experiment. Anaesthesia was induced by intraperitoneal injection of Ethylurethane (0.9 g.kg$^{-1}$). The guinea pig was fixed on a thermostated operation table, monitored by a thermosensible detector rectally applied. Heating was carried out by the operation table itself and two lamps located above the animal.

In the evacuated colon descendens two segments were localised. In both segments, later called sample- and reference segment small measuring electrodes were fixed, displaying continuously the absolute temperature until 0.001°C. Two other electrodes linked by a Wheatstone bridge connection were equally located in the two segments. They measure continuously the temperature difference between the two segments.

Small temperature fluctuations of the anaesthetised animal do not affect the measurements. In both segments a smooth catheter (I.D. 0.3 mm) was fixed, winded several times in the intestinal cavity. In this catheters 100 $\mu$l of the reference- and sample solution was injected.

The intestinal cavity was sealed again. This set up enables the injection of solutes at body temperature. The animal was thermally isolated by gauge. After ten minutes, the wheatstone bridge was equilibrated. An injection of 100 $\mu$l air evacuates the catheters so injecting the solutions at perfect body temperature.

#### 2.2. Calibration of the electrodes

The electrodes displaying the absolute temperature were calibrated in a thermostatically controlled water bath between 36.0°C and 38.0°C. The measured resistance differences in n were logarithmic. The electrodes measuring the temperature difference were calibrated by two thermostatically controlled water baths with temperature differences between 0.0°C and 2.0°C. The rectal temperature was directly monitored in °C.

2.3. Used solutions

* reference solution:

- Krebsolution - TRIS.HCl buffer 0.1 M pH 8.0 (9/1) osmotic pressure equals 0.9 % Sodium Chloride

* Sample solutions :
- Rhein saturated in reference solution. Concentrations : 9.52 x 10-5 g/100 ml
- 1, 8 Dihydroxyanthraquinone saturated in reference solution, concentration : $6.41 \times 10^{-7}$ g/100 ml.

The addition of the anthraquinones did not affect the osmotic pressure.

3. Results

After filling up the two colonic segments with reference solution, the temperature of both the reference- and the sample compartment remained stable during the whole experimental set-up. The temperature difference between the two segments is minimal and never exceeds -0.031˚C.

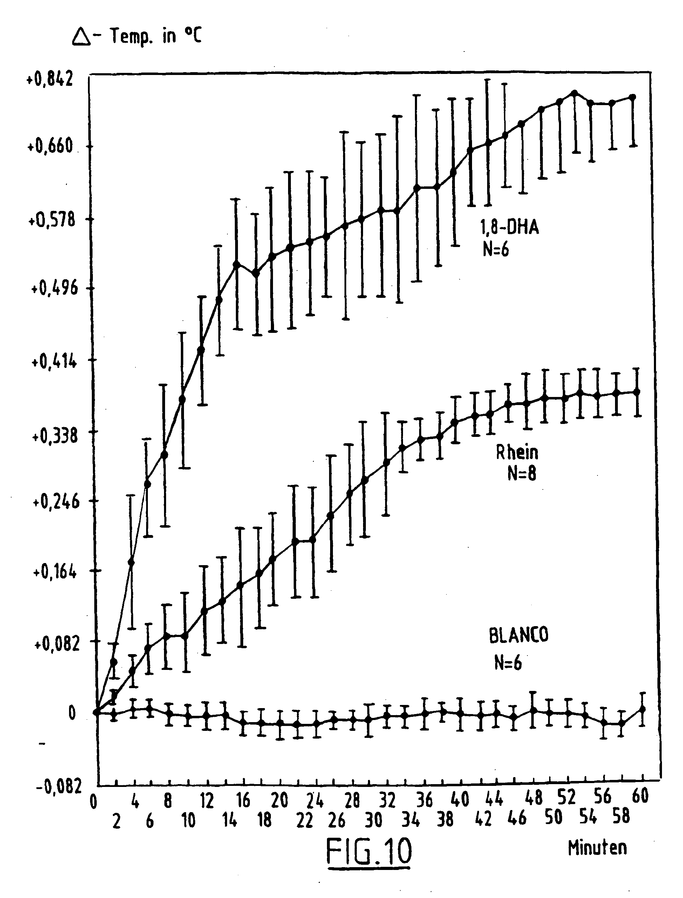

The presence of an anthraquinone containing solution results in a temperature rise of 0.36˚C for rhein and even 0.70˚C for 1,8-DHA as is shown in Fig. 10.

Although, due to lower solubility, the injected 1,8-DHA concentration is considerably lower vs. the rhein concentration (0.64 $\mu$g vs. 95.2 $\mu$g), the effect is considerably higher. This can be explained by the higher uncoupling effect of 1,8-DHA. All recorded differences are statistically significant.

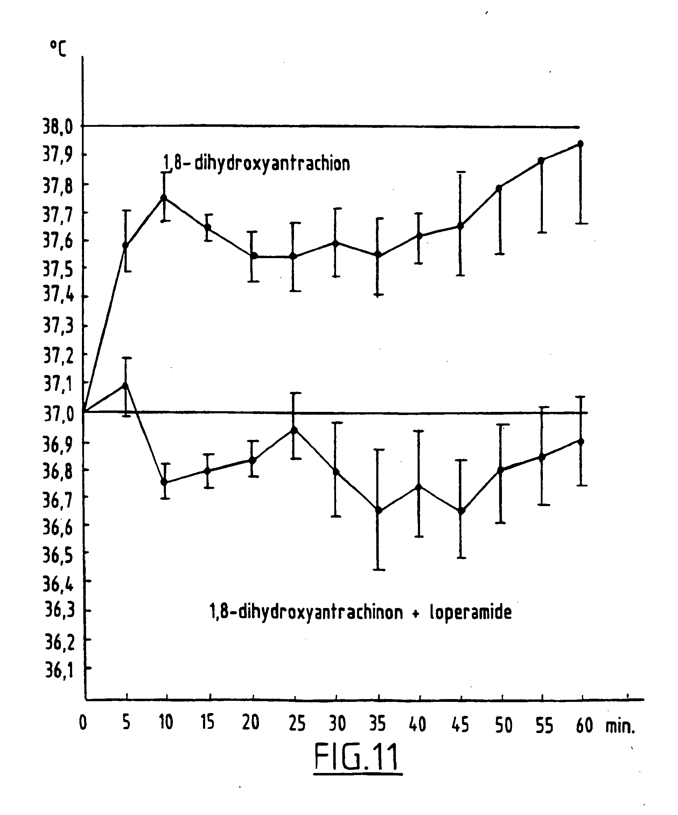

Previous administration of loperamide antagonises the temperature rise following DHA administration as displayed in Fig. 11. Also other tested mitochondrial uncouplers as DNP and salicylic acid show the same results.

4. Discussion

The measured temperature increase due to anthraquinone addition is remarkable, especially as additional heat dissipation by intestinal veins and arteries is expected. It should be pointed out that the recorded temperature rise is not due to a local vasodilatation resulting in an increased blood flow. Actually, the recorded temperatures are even significantly higher then the internal temperature of the test animals. Also here, the direct or indirect involvement of mitochondria in the permeability changes is evident. The difference in activity between rhein and 1,8-DHA is also reflected by their difference in mitochondrial uncoupling activity as demonstrated from Fig. 10.

**EXAMPLE 6**

The influence of Rhein and loperamide on the ATP content of a monocellular layer of mucosal cells.

1. Introduction

It was found that a significant ATP decrease was measured in the monocellular mucosal tissue accompanying an increased permeability. However, after a certain exposure-time the mitochondria reproduce ATP. This demonstrates the relative harmlessness of the mitochondrial therapy.

2. Materials and methods

The ATP content was measured in the monocellular mucosal layer of the guinea-pig colon.

2.1. Operation procedure

Guinea pigs with a weight (mean $\pm$ 400 g) were fasted 12 hours before the experiments. Anaesthesia was induced by intraperitoneal injection of Ethylurethane (0.9 g.kg$^{-1}$).

Colon segments with a physiological length of 5 branches of the superior mesenteric vein were ligated. The segment was filled with 4.0 ml of the solution to be tested. The intestinal cavity was immediately sealed again. After the experiment the colon segment was removed and submerged in liquid Nitrogen. The gut specimen was transferred to the precooled lyophylisator (-80˚C, sample -20˚C) and lyophilised during 36 hours. Microscopically, the mono cellular mucosa layer was scraped off. One segment, proceeding this way normally yield 1.5 - 2.0 mg lyophilised mucosa cells. 1.000 mg mucosa was weighted carefully on a microbalans and homogenised with 100.00 mg lactose. The ATP level was measured on 3.000 mg of this mixture.

## 2.2. ATP measurement

ATP was measured following the firefly-bioluminescence system on a LKB Wallac 1250, fitted with a 1250 display.

Required reagents

-   Luciferin - Luciferase solution: LKB
    10.0 mg lyophilised powder was solved in 10.0 ml bidistilled water

    -   Standardised ATP solution: $10^{-5}$ M
    -   Buffer solution: TRIS.HCl 0.1M, pH 7.75 - 0.372 g $Na_2EDTA$ was added for 0.5 1 solution

## 2.2.1. calibration procedure

For the blank experiment 200 $\mu$l Luciferine-Luciferase solution was mixed with 500 $\mu$l TRIS-EDTA buffer solution. The light emission was measured following the addition of standard amounts of ATP.

| Amount of injected ATP in $10^{-5}$M | h$\nu$ emission in Mv |
|---|---|
| 10 | 436 |
| 20 | 851 |
| 30 | 1251 |
| 40 | 1647 |
| 50 | 2027 |
| 60 | 2346 |

Studies point out that the ATP decay is not linear but exponential following the formula:

$$\frac{\ln\ (y/a)}{b} = x$$

with

y:    numerical value in mV
x:    decay in mV/minute
a :   341.73
b :   0.29

After correction for the background the following values were obtained:

| Amount of injected ATP in $10^{-5}$M | h$\nu$ emission in Mv - background |
|---|---|
| 0.990 | 431 |
| 1.966 | 846 |
| 2.913 | 1246 |
| 3.846 | 1642 |

(continued)

| Amount of injected ATP in $10^{-5}$M | hν emission in Mv - background |
|---|---|
| 4.762 | 2022 |
| 5.660 | 2341 |

The recovered calibration curve was perfectly linear yielding the following statistical parameters:

$r^2$ (correlation coefficient) :    0.99998
b :    2.097
a :    $4.3036 \times 10^{-9}$

## 2.2.2. Calibration procedure in the presence of Rhein

While it was clear that Rhein exhibited an inhibiting effect on the Lucerferin -Luciferase complex, the calibration curve was made in the presence of the concentration Rhein, recovered in the experiment.

3.000 mg of the mucosa/lactose mixture was suspended in 800 μl TRIS-EDTA buffer solution. After a sonication of 30 minutes at 60˚C, the mixture was shaked for two hours in order to hydrolyse all present ATP. 200 μl Luciferin-Luciferase solution was added. Known quantities of ATP were added. The following results were noted:

| Amount of injected ATP in $10^{-7}$M | hν emission in Mv (after correction) |
|---|---|
| 0.990 | 356 |
| 1.961 | 648 |
| 2.913 | 972 |
| 3.846 | 1320 |
| 4.762 | 1608 |
| 5.660 | 1892 |

Regression analysis yielded following results:

$r^2$ (correlation coefficient) :    0.9990
b :    - 0.821
a :    $3.368 \times 10^{-9}$

## 2.2.3. Test on homogenisation

In order to evaluate the homogenisation process (1.000 mg mucosa in 100.0 mg lactose) several samples were analysed for their ATP content

| Weighted amount (mg) | hν emission in Mv (after correction) |
|---|---|
| 2.996 | 435 |
| 3.022 | 427 |
| 3.020 | 412 |
| 3.022 | 402 |
| Mean | 419 |
| Standard deviation: | 15 |

(continued)

| Weighted amount (mg) | hν emission in Mv (after correction) |
|---|---|
| Variability | 4% |

These values confirmed an efficient homogenisation procedure.

3. Results

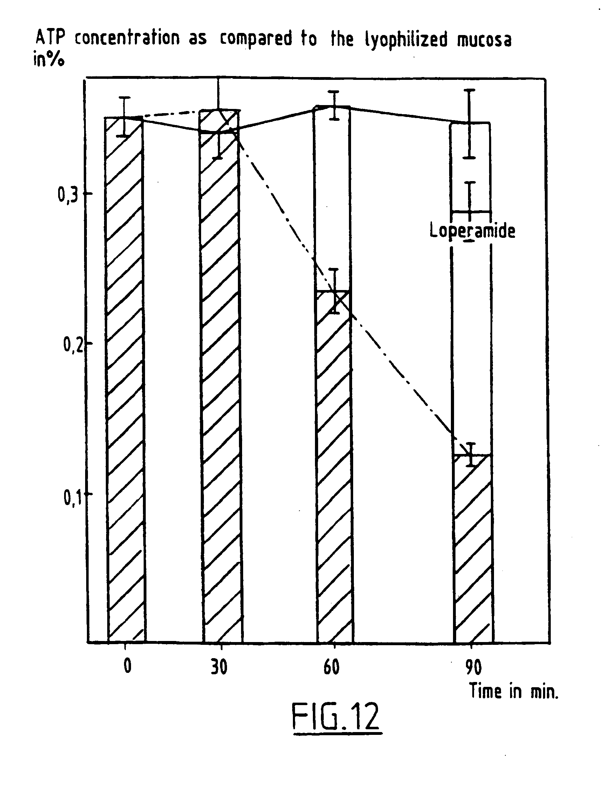

Fig. 12 shows the ATP concentration in lyophilised mucosa (in %) in function of time (black line). The shaded line represents the evolution of the ATP content in the presence of anthraquinone. At time 90 minutes the ATP concentration is displayed in the presence of anthraquinone and loperamide.

4. Discussion

The presence of 3.76 mg rhein in a ligated colonic segment results in a significant decrease of the available ATP concentration in the mucosal cells. The first effect is noted after 30 minutes. After 60 minutes only 67.1 % of the initial ATP concentration can be recovered. After 90 minutes only 35.9 % remains. Most probably 30 minutes is the latency time, required for the rhein molecule to permeate across the cellular membrane and reach the mitochondrial region.

**EXAMPLE** 7

Resorption of a non-resorbable drug

1. Introduction

Experiments where carried out in order to enhance the resorption of non resorbable drugs. The experiments were performed with insulin while this molecule cannot be administrated orally because destruction in the stomach is evident. Enteric coating of the administration form avoids the stomachal break-down allowing the release in the intestine. However, due to its high molecular weight, Insulin is not resorbed. Even rectal administration is not possible for the same reason. Insulin is a good working tool while resorption readily results in an easily measurable blood glucose level. Glucose blood values were compared versus the effects of an intramuscular Insulin injection.

2. Materials and methods

In order to evaluate the transmucosal colonic absorption of Insulin, several experimental set ups have been used.
Experiments have been carried out on Rats (Wistar), Male ($\pm$ 150 animals), Guinea pigs, Male ($\pm$ 600 animals), Rabbits, Male ($\pm$ 4.5 kg) ($\pm$ 400 animals) and Dogs (Beagles) Male ($\pm$ 32 kg) (6 animals).
The resorption of Insulin has been monitored by the lowering of the blood glucose level versus an intramuscular injection of an equivalent dose. Glucose was monitored while the determination is simple and inexpensive. However, on several experiments the Insulin level was measured by RIA (Radio Immuno Assays) methods.

2.1. Experimental set up 1

Initially the resorption of Insulin was monitored following a perfusion on anaesthetised animals. Anaesthesia was induced by abdominal injection of Ethylurethane (0.9 g/kg).
The circulating solution was principally isotonic Sodium chloride (physiological saline) to which the appropriate Insulin was added in order to obtain the required concentrations.
The used Insulin was ACTRAPID™ (Novo Nordisk); Human Biosynthetic Insulin.
The perfusion itself was executed on a new developed manner in order to allow a perfusion without an operation. Blood was sampled on the jugular vein.

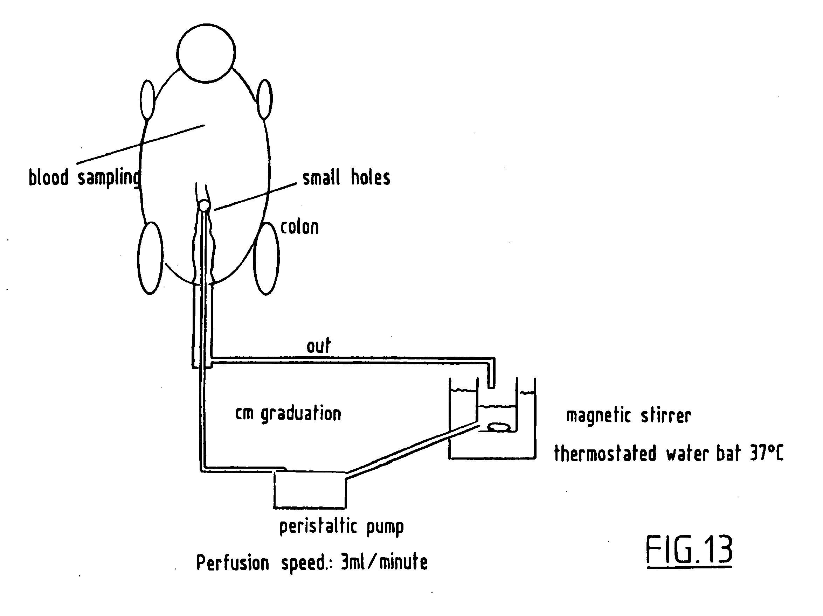

Figure 13 shows the scheme of the developed perfusion experiment.
The device used allows an easy rectal application. It allows an exact positioning and an identical length of perfused colon. It was found that the perfused solution did not leak out of the perfused colonic segment.
The total circulating fluid was 8 ml. As tubing Isoversinic was used. At intervals 50 $\mu$l blood was sampled and used for the glucose determinations. These perfusion experiments were carried out on guinea pigs and rats. These experiments revealed that virtually 0% Insulin is resorbed resulting in an unmodified blood glucose level. However, addition of Rhein or Indomethacin (total concentration 0.5 mg) was able to allow the Insulin absorption to $\pm$ 44 % of the effect obtained after an intramuscular injection.

Two reasons were found:

1. An intramuscular injection can be considered as a bolus administration while the perfusion is a continue process. The pharmacokinetic is totally different.
2. It is possible that, although the addition of a mitochondrial uncoupler opens the paracellular route a physical driving force was lacking, forcing the Insulin from the mucosal side to the serosal one.

2.2. Experimental set up 2

Frequent blood sampling in small animals only allows the collection of small blood volumes. This interferes with the chemical determination. Therefore experiments were carried out on rabbits. Male rabbits were immobilised in a metal cage allowing free access to the anal region and the auricular arteries of both ears. In these experiments narcosis was not essential. The sampled blood volume was 0.5 ml.

Small hollow suppositories were made with dimensions $\pm$ 2 cm, $\phi$ 0.5 cm. The suppositories were filled with ACTRAPID™ (Novo Nordisk) 100 I.U./ml in the appropriate dilution with physiological saline. The anal region was secured with adhesive tape. It could be stated that Insulin has a very low penetration rate. However, incorporation in the suppository mass of a mitochondrial uncoupler as 1,8 dihydroquiantraquine 3 mg, Rhein 3 mg, Indomethacin 1.5 mg causes a substantial resorption of Insulin. The blood glucose amount falls to $\pm$ 50 % of the effect obtained after intramuscular injection of an identical dose.

The following concentrations were used 0.1 I.U./kg body weight, 0.5 I.U./kg body weight, 1.0 I.U./kg body weight.

As the content of the suppository was approximately 0.6 ml, 450 $\mu$l (at the highest dose) of the Insulin solution was mixed with 150 $\mu$l normal saline.

It was found that the osmotic pressure was an excellent driving force. For these experiments Insulin from bovine pancreas (activity 41.67 $\mu$g = 1 I.U.) Fluka was solved in 0.6 ml bidistilled water. The experiment was carried out as outlined above. It was stated that the presence of this driving force results in a blood glucose decrease statistically equivalent to the intramuscular injection for the maximum peak effect and the time required to reach it and the length of the effect (A.U.C. = Area Under the Curve).

It should be noted, that the amino acid composition and physical structure of mammalian Insulin varies only slightly from one species to the next, except for the rat and the guinea pig which have larger structural differences.

As a clear effect was seen both by rats and guinea pigs the use of bovine pancreas Insulin was thought to be effective in rabbits. The experiments with dogs (Beagles) exhibited the same results. However, the dogs were anaesthetised.

3. Results

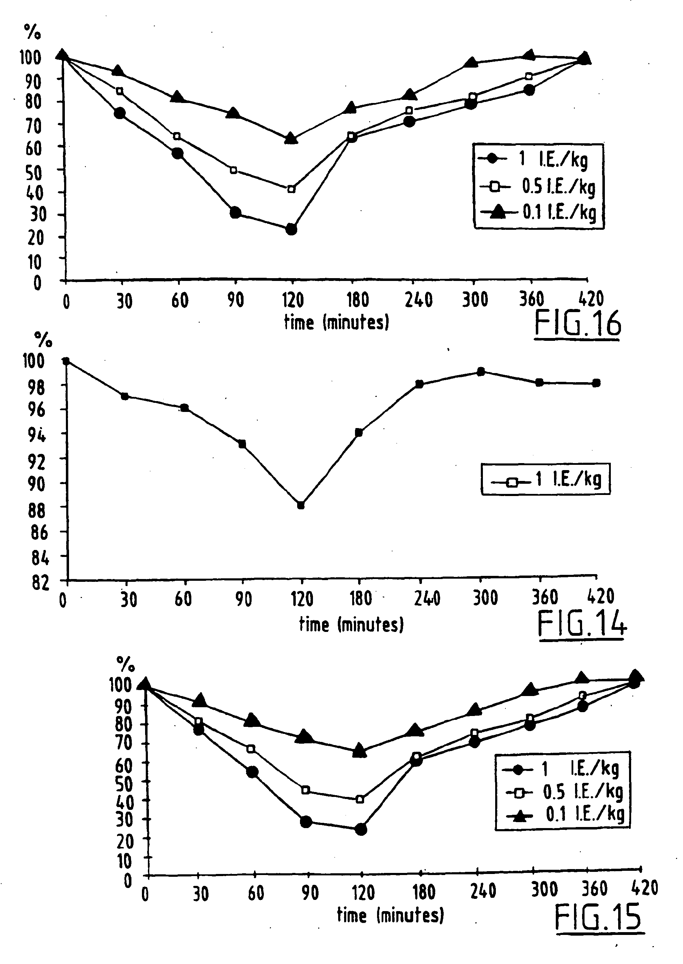

Rectal resorption of insulin is virtually nil in guinea pigs, rats and rabbits as is shown in Fig. 14.

Fig. 15 shows the blood glucose content after intramuscular insulin injection. Even in the lowest dose the blood glucose content after 120 minutes amounts to $\pm$ 60% of the initial value.

The aim of the study was to obtain a resorption pattern identical to intramuscular injection. However, it was found that although the tight junctions of the paracellular route opened, a driving strength forcing the molecule from the intestinal lumen trough the tight junction was lacking. Dissolution or dilution of the insulin in distilled water delivered the osmotic force, necessary to enhance a fast and easy entry.

Fig. 16 shows the results after dissolution or dilution of the carrier composition of insulin and the uncoupling agent indomethazine with distilled water. The obtained peak effect was 120 minutes and identical to an intramuscular injection. The finally obtained blood glucose level is identical to an intramuscular injection ($\pm$ 20% of the initial value). Also the duration of the effect is identical.

4. Conclusion

This experiment demonstrates that it is possible to administer a non-resorbable substance, insulin, rectally with a similar effectiveness as intramuscular injection.

**Claims**

1. Carrier composition for transporting a biologically active substance into the body of a patient comprising:

   a) at least one mitochondrial uncoupler, wherein said mitochondrial uncoupler is an agent leading to a decrease or termination of ATP production in mitochondria, selected from the group consisting of the non steroidal anti inflammatory drugs (NSAIDS), salicylic add and its different salts, arylacetic acid derivatives, arylpropionic add derivatives, indole derivatives, fenamates, oxicams and phenylbutazone, or the polyphenols like dihydroxyanthraquinones, oxyphenisatin, phenolphthalein, or bile salts and their derivatives, as well as several oils or fatty acids, like ricinus oil or ricinoleic add;
   b) one or more osmotic solution(s) for controlling the direction of transport of the carrier composition into the body, wherein said osmotic solution(s) is (are) hypotonic; and
   c) one or more biologically active substance(s).

2. Carrier composition as in claim 1, wherein the biologically active substance is selected from the group consisting of peptide hormones, such as calcitonin, secretin, vasopressin, insulin, angiotensin, felypressin, thyrotropin releasing hormone, gonadotropin releasing hormone, corticotropin, prolactin, somatotropin, thyrotropin, luteinizing hormone (LH), callicrein, parathyrin, glucagons, oxytocin, gastrin and serum gonadotropin, or polysaccharides, such as heparin sodium heparin calcium and chondroitin sulfate or cosmetically active substances, like collagen.

3. Carrier composition as claimed in any of Claims 1 to 2 having the form of sublingual tablets, hollow suppositories or capsules filled with the composition, nose drops, sprays, aerosols, unguents, ointments, gels, liquids, or oils.

4. Carrier composition as claimed in any of Claims 1 to 2 having a rectal, vaginal, sublingual, nasal or oral administration form.

5. Carrier composition as claimed in any one of the claims 1 to 4 for use as a diagnostic, therapeutical, prophylactic or cosmetic composition.

6. Use of a carrier composition as claimed in any one of Claims 1 to 4 for the preparation of a diagnostic, therapeutical, prophylactic or cosmetic composition for active opening of tight junctions.

**Patentansprüche**

1. Trägerzusammensetzung zum Transportieren einer biologisch aktiven Substanz in den Körper von einem Patienten, umfassend:

   a) mindestens einen mitochondrialen Entkoppler, wobei der mitochondriale Entkoppler ein Wirkstoff ist, der zu einer Abnahme oder Beendigung der ATP-Produktion in Mitochondrien führt, der aus der Gruppe ausgewählt ist, die aus den nichtsteroidalen Antiphlogistika (NSAID), Salizylsäure und seinen verschiedenen Salzen, Derivaten der Arylessigsäure, Derivaten der Arylpropionsäure, Indolderivaten, Fenamaten, Oxicamen und Phyenylbutazonen oder den Polyphenolen wie Dihydroxyanthraquinonen, Oxyphenisatin, Phenolphthalein, oder den Gallensalzen und ihren Derivaten, sowie verschiedenen Ölen oder Fettsäuren, wie Rizinusöl oder Rizinolsäure besteht.
   b) eine oder mehrere osmotische Lösung(en) zur Kontrolle der Richtung des Transportes von der Trägerzusammensetzung in den Körper, wobei die osmotische Lösung/die osmotischen Lösungen hypotonisch ist/sind; und
   c) eine oder mehrere biologisch aktive Substanz(en).

2. Trägerzusammensetzung nach Anspruch 1, wobei die biologisch aktive Substanz ausgewählt ist aus der Gruppe, die aus Peptidhormonen, wie Calcitonin, Sekretin, Vasopressin, Insulin, Angiotensin, Felypressin, Thyrotropin-Releasing-Hormon, Gonadotropin-Releasing-Hormon, Corticotropin, Prolactin, Somatotropin, Thyrotropin, Luteinizing-Hormon (LH), Kallikrein, Parathormon, Glucagonen, Oxytocin, Gastrin und Serum-Gonadotropin oder Polysacchariden, wie Heparin-Natrium, Heparin-Calcium und Chondroitinsulfat oder kosmetisch aktiven Substanzen, wie Kollagen, besteht.

3. Trägerzusammensetzung nach einem der Ansprüche 1 oder 2, welche die Form von sublingualen Tabletten, hohlen Suppositorien oder Kapseln, die mit der Zusammensetzung gefüllt sind, Nasentropfen, Sprühmitteln, Aerosolen, Salben, Wundsalben, Gelen, Flüssigkeiten oder Ölen aufweist.

4. Trägerzusammensetzung nach einem der Ansprüche 1 oder 2, welche eine rektale, vaginale, sublinguale, nasale oder orale Verabreichungsform aufweist.

5. Trägerzusammensetzung nach einem der Ansprüche 1 bis 4, zur Verwendung als eine diagnostische, therapeutische, prophylaktische oder kosmetische Zusammensetzung.

6. Verwendung von einer Trägerzusammensetzung nach einem der Ansprüche 1 bis 4, für die Zubereitung einer diagnostischen, therapeutischen, prophylaktischen oder kosmetischen Zusammensetzung zum aktiven Öffnen von Zell-Zell-Kontakten (tight junctions).


**Revendications**

1. Composition de transport destinée à transporter une substance biologiquement active dans le corps d'un patient, qui comprend :

   a) au moins un découpleur mitochondrial, ledit découpleur mitochondrial étant un agent conduisant à une diminution ou un arrêt de la production d'ATP dans les mitochondries, choisi dans le groupe constitué par les anti-inflammatoires non stéroïdiens (AINS), l'acide salicylique et ses différents sels, les dérivés de l'acide arylacétique, les dérivés de l'acide arylpropionique, les dérivés indole, les fénamates, les oxicanes et le phénylbutazone, ou les polyphénols tels que les dihydroxyanthraquinones, l'oxyphénisatine, la phénolphtaléine, ou les sels biliaires et leurs dérivés, ainsi que plusieurs huiles ou acides gras, tels que l'huile de ricin ou l'acide ricinoléique ;
   b) une ou plusieurs solution(s) osmotique(s) destinée(s) à contrôler la direction de transport de la composition de transport dans le corps, ladite ou lesdites solution(s) osmotique(s) étant hypotonique(s) ; et
   c) une ou plusieurs substance(s) biologiquement active(s).

2. Composition de transport selon la revendication 1, dans laquelle la substance biologiquement active est choisie dans le groupe constitué par les hormones peptidiques, telles que la calcitonine, la sécrétine, la vasopressine, l'insuline, l'angiotensine, la félypressine, l'hormone de libération de la thyrotropine, l'hormone de libération de la gonadotropine, la corticotropine, la prolactine, la somatotropine, la thyrotropine, l'hormone lutéinisante (LH), la callicréine, la parathyrine, les glucagons, l'oxytocine, la gastrine et la gonadotropine sérique, ou les polysaccharides, tels que l'héparine sodique, l'héparine calcique et la chondroïtine sulfate ou des substances actives sur le plan cosmétique, telles que le collagène.

3. Composition de transport selon l'une quelconque des revendications 1 à 2, qui a la forme de comprimés sublinguaux, de suppositoires creux ou de gélules remplies de la composition, de gouttes nasales, de sprays, d'aérosols, de pommades, d'onguents, de gels, de liquides, ou d'huiles.

4. Composition de transport selon l'une quelconque des revendications 1 à 2, qui a une forme d'administration rectale, vaginale, sublinguale, nasale ou orale.

5. Composition de transport selon l'une quelconque des revendications 1 à 4, destinée à être utilisée en tant que composition diagnostique, thérapeutique, prophylactique ou cosmétique.

6. Utilisation d'une composition de transport selon l'une quelconque des revendications 1 à 4, pour la préparation d'une composition diagnostique, thérapeutique, prophylactique ou cosmétique pour l'ouverture active des jonctions serrées.

FIG.2

FIG.1

FIG.3

FIG.4

colon segment     kidneys     urine

% of injected radioactivity

FIG.5

Colon     Kidneys     Urine

ANTAGONISTIC EFFECT OF MORPHINE IN GUINEA-PIG PERFUSED COLON

FIG.6

FIG.7

FIG.8

ADP    + Pi  ·······························▶ ATP + Heath
       Metabolism of the fuel Molecules

                                          Cellulair demand    Active ion transportation

                                                    ADP + Pi

## FIG.9

EP 0 938 338 B1

△ - Temp. in °C

1,8-DHA
N=6

Rhein
N=8

BLANCO
N=6

FIG.10

Minuten

FIG.11

ATP concentration as compared to the lyophilized mucosa in%

FIG.12

blood sampling

small holes

colon

out

cm graduation

magnetic stirrer

thermostated water bat 37°C

peristaltic pump

Perfusion speed.: 3ml/minute

FIG.13

EP 0 938 338 B1

FIG.16

FIG.14

FIG.15